(19)

**Europäisches Patentamt**
**European Patent Office**
**Office européen des brevets**

(11) **EP 2 232 255 B1**

(12) **EUROPÄISCHE PATENTSCHRIFT**

(45) Veröffentlichungstag und Bekanntmachung des Hinweises auf die Patenterteilung:
**22.02.2012 Patentblatt 2012/08**

(21) Anmeldenummer: **08871618.8**

(22) Anmeldetag: **20.12.2008**

(51) Int Cl.:
*G01N 33/00* (2006.01)

(86) Internationale Anmeldenummer:
**PCT/EP2008/010973**

(87) Internationale Veröffentlichungsnummer:
**WO 2009/092429 (30.07.2009 Gazette 2009/31)**

(54) **VERFAHREN ZUR ERMITTLUNG DER RUßOXIDATIONSRATE VON IN EINEM PARTIKELFILTER ZURÜCKHALTENEM RUß**

METHOD FOR DETERMINING THE PARTICULATE OXIDATION RATE OF PARTICULATES RETAINED IN A PARTICULATE FILTER

PROCÉDÉ POUR DÉTERMINER LE TAUX D'OXYDATION DE LA SUIE RETENUE DANS UN FILTRE À PARTICULES

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **23.01.2008 DE 102008005640**

(43) Veröffentlichungstag der Anmeldung:
**29.09.2010 Patentblatt 2010/39**

(73) Patentinhaber: **Daimler AG**
**70327 Stuttgart (DE)**

(72) Erfinder:
- **LAHR, Jochen**
  **70190 Stuttgart (DE)**
- **PAULS, Rainer**
  **71665 Vaihingen (DE)**

(74) Vertreter: **JENSEN & SON**
**366-368 Old Street**
**London**
**EC1V 9LT (GB)**

(56) Entgegenhaltungen:
**EP-A- 0 869 359      EP-A- 1 174 712**
**EP-A- 1 628 133      DE-A1- 4 402 850**
**FR-A- 2 864 146**

- **KANDYLAS I.P.; HARALAMPOUS O.A.; KOLTSAKIS G.C.: 'Diesel Soot Oxidation with NO2: Engine Experiments and Simulations' IND. ENG. CHEM. RES. Bd. 41, Nr. 22, 20 September 2002, DOI: 10.1021/IE020379T, Seiten 5372 - 5384**
- **KANDYLAS, I P; HARALAMPOUS, O A; KOLTSAKIS, G C: "Diesel Soot Oxidation with NO2: Engine Experiments and Simulations", IND. ENG. CHEM. RES., vol. 41, no. 22, 20 September 2002 (2002-09-20), pages 5372-5384, DOI: 10.1021/ie020379t**

**Beschreibung**

Die Erfindung betrifft ein Verfahren zur Berechnung einer Rußoxidationsrate von in einem Partikelfilter in einem Abgasstrang einer Verbrennungseinrichtung, insbesondere einer Brennkraftmaschine, zurückgehaltenem Ruß.

In der DE 10 2005 049 655 A1 ist auf die bedeutende Rolle von Stickstoffdioxid ($NO_2$) als Reaktionspartner in einer Vielzahl von Reaktionen bei einer Nachbehandlung von Brennkraftmaschinenabgas hingewiesen. Andererseits ist $NO_2$ eine toxische Abgaskomponente, deren Freisetzung möglichst vermieden werden sollte. Dementsprechend ist vorgeschlagen, den $NO_2$-Gehalt im Abgas geeignet einzustellen. Hierzu wird die Aktivität eines im Abgasstrang angeordneten Katalysators mit Fähigkeit zur Oxidation von Stickstoffmonoxid (NO) zu $NO_2$ beeinflusst, was durch Variation des Anteils an Stoffen geschieht, welche mit der besagten Oxidationsreaktion konkurrieren. Zur Kontrolle des Verfahrens wird unter anderem eine sensorische $NO_2$-Bestimmung vorgeschlagen. Allerdings sind derzeit übliche Sensoren zur selektiven Bestimmung von $NO_2$ für einen praktischen Einsatz im Abgas von Verbrennungseinrichtungen nicht oder nur schlecht geeignet. Übliche, zur Messung der Konzentration von Stickoxiden (NOx) im Abgas eingesetzte Sensoren weisen vielmehr eine Empfindlichkeit sowohl gegenüber NO als auch gegenüber $NO_2$ auf, so dass zwischen diesen beiden Stickstoffoxiden nicht unterschieden werden kann. Andererseits ist es gemäß dem vorstehend geschilderten Sachverhalt in vielerlei Hinsicht wünschenswert, ein den $NO_2$-Gehalt im Abgas repräsentierendes Signal verfügbar zu haben.

EP 0 869 359 A2 offenbart eine Vorrichtung zur Ermittlung niedriger NOx-Konzentrationen, bei der zwei NOx-Sensoren und ein dazwischen angeordneter Oxidationskatalysator in einer gemeinsamen Kammer angeordnet sind, welcher ein konstanter Luftstrom zugeführt wird. Der Oxidationskatalysator wird auf einer konstanten Temperatur gehalten und kann das Konzentrationsverhältnis von NO und $NO_2$ auf dem thermodynamischen Gleichgewichtswert halten. Die von der NOx-Konzentration abhängigen Ausgangssignale der NOx-Sensoren werden einer Auswerteinheit zugeführt und durch eine Berechnung können sowohl die NO- als auch die $NO_2$- und damit auch die NOx-Konzentration bestimmt werden.

FR 2 864 146 A1 offenbart ein Verfahren zur Echtzeitbestimmung der Rußbeladung eines Partikelfilters, dem ein Oxidationskatalysator vorgeschaltet sein kann. Hierzu werden die Außentemperatur, die Sauerstoffkonzentration und die NOx-Konzentration eingangsseitig des Partikelfilters erfasst und durch Differenzbildung von Rußemissionsrate des Motors und der nach Gesetzen der Kinetik ermittelten Rußverbrennungsrate die Rußbeladung des Partikelfilters errechnet. Zur Erfassung der NOx-Konzentration kann ein NOx-Sensor vorgesehen sein.

DE 44 02 850 A1 offenbart ein Motorüberwachungssystem und eine zugehörige Vorgehensweise um diverse Motorfunktionen bzw. Komponenten eines zugeordneten Abgasreinigungssystems zu überwachen. Unter anderem wird die Überwachung eines NOx-Katalysators mittels eines ersten, vorgeschalteten und eines zweiten, nachgeschalteten NOx-Sensors beschrieben.

Kandylas et al. beschreiben in dem Fachartikel "Diesel Soot Oxidation with NO2: Engine Experiments and Simulations" (Ind. Eng. Chem. Res., Bd. 41, Nr. 22, 20.9.1002, Seiten 5372-5384) ein Verfahren zur Berechnung einer Oxidationsrate von Ruß. Hierzu werden Differenzen ΔNO und ΔCO der vor und hinter dem Partikelfilter auftretenden Konzentrationen von NO und CO mittels Gas-Analysatoren, denen eine Teilstrom des Abgases zugeführt wird, ermittelt und entsprechend ausgewertet. Aus den Differenzen wird unter Berücksichtigung der bekannten Reaktionsgleichung der Rußoxidation weiter die Rußoxidationsrate rechnerisch ermittelt.

Aufgabe der Erfindung ist es, ein Verfahren anzugeben, welches auf einfache und in einem praktischen Fahrbetrieb eines Kraftfahrzeugs durchführbare Weise die Berechnung einer Oxidationsrate von in einem Partikelfilter abgelagertem Ruß ermöglicht.

Diese Aufgabe wird durch ein Verfahren mit den Merkmalen des Anspruchs 1 gelöst.

In dem erfindungsgemäßen Verfahren wird einer Stickstoffdioxidkonzentration und/oder ein Konzentrationsverhältniss von Stickstoffdioxid und Stickstoffmonoxid in einem Abgasstrang einer Brennkraftmaschine mittels eines in Bezug auf Stickstoffmonoxid und Stickstoffdioxid empfindlichen Stickoxidsensors ermittelt, welcher als einziges in Bezug auf Stickoxide relevantes Ausgangssignal ein Stickoxid-Ausgangssignal bereitstellt, das mit einer durch die Summe der Konzentrationen von Stickstoffmonoxid und Stickstoffdioxid repräsentierten Stickoxidgesamtkonzentration korreliert. Dabei wird zur Ermittlung der Stickstoffdioxidkonzentration und/oder des Konzentrationsverhältnisses von Stickstoffdioxid und Stickstoffmonoxid das Stickoxid-Ausgangssignal eines ersten Stickoxidsensors, der stromauf eines im Abgasstrang angeordneten oxidationskatalytisch wirksamen Abgasbehandlungselements mit Fähigkeit zur Umsetzung von Stickstoffmonoxid zu Stickstoffdioxid angeordnet ist, mit dem Stickoxidausgangssignal eines zweiten Stickoxidsensors, der stromab des oxidationskatalytisch wirksamen Abgasbehandlungselements angeordnet ist, verglichen. Aus der ermittelten Stickstoffdioxidkonzentration und/oder aus dem ermittelten Konzentrationsverhältnis von Stickstoffdioxid und Stickstoffmonoxid wird die Rußoxidationsrate von in einem dem zweiten Stickoxidsensor nachgeschaltetem Partikelfilter zurückgehaltenem Ruß berechnet. Dabei werden bei einer Bezugnahme auf Stickstoffmonoxid bzw. Stickstoffdioxid die Bezeichnungen "NO" und "$NO_2$" verwendet, während bei einem summarischen Bezug auf NO und $NO_2$ umfassende Stickstoffoxide die Bezeichnung "NOx" verwendet oder allgemein von "Stickoxid" gesprochen wird.

Insbesondere unter Kenntnis oder Annahme gewisser Randbedingungen am Ort des ersten Stickoxidsensors wie beispielsweise einer vorgegebenen NOx-Konzentration und/oder eines vorgegebenen Konzentrationsverhältnisses von NO und $NO_2$ und/oder einer vorgegebenen $NO_2$-Konzentration, lässt sich durch einen Vergleich der Ausgangssignale der beiden Stickoxidsensoren das Konzentrationsverhältnis von NO und $NO_2$ und/oder die $NO_2$-Konzentration insbesondere am Ort des zweiten Stickoxidsensors ermitteln. Dies ist insofern von Bedeutung, als sich ausgehend von einer meist vernachlässigbaren $NO_2$-Konzentration im unmittelbar von der Verbrennungseinrichtung abgegebenen Abgas das Konzentrationsverhältnis von NO und $NO_2$ und/oder die $NO_2$-Konzentration mit dem Passieren von im Abgasstrang angeordneten Abgasbehandlungselementen im Allgemeinen ändert. Dabei kann die NOx-Konzentration unverändert bleiben. Eine solche Änderung kann auch ohne Einwirkung einer Abgasbehandlungskomponente allein infolge der vorgegebenen thermodynamischen Randbedingungen eintreten. Beispielsweise erfolgt bei Anwesenheit von freiem Sauerstoff eine natürlich ablaufende Oxidation von NO zu $NO_2$.

Das erfindungsgemäße Verfahren kann mit Vorteil bei Verbrennungsmotoren von Kraftfahrzeugen, insbesondere bei wenigstens zeitweise mit Luftüberschuss betriebenen Verbrennungsmotoren, wie Diesel- oder Mager-Ottomotoren angewendet werden. Dementsprechend kann es sich bei dem oxidationskatalytisch wirksamen Abgasbehandlungselement um einen Oxidations-, Denox- oder Dreiwegekatalysator oder um einen katalytisch beschichteten Partikelfilter handeln. Eine Fähigkeit zur Beeinflussung der $NO_2$-Konzentration und/oder des Konzentrationsverhältnisses von NO und $NO_2$ kann durch eine dem Abgasbehandlungselement unmittelbar zuzuschreibende Eigenschaft oder durch eine von mit dem Abgas zugeführten Stoffen oder durch Ablagerungen hervorgerufene Eigenschaft bewirkt sein. Beispielsweise kann in einem von Abgas durchströmten Partikelfilter auf diesem abgelagerter Ruß $NO_2$ zu NO reduzieren oder es kann eine Oxidation von NO zu $NO_2$ in einem von Abgas durchströmten Oxidationskatalysator erfolgen. Ferner hat bei vielen Abgasnachbehandlungsverfahren, wie beispielsweise bei der selektiven katalytischen NOx-Reduktion mittels Ammoniak, Wasserstoff oder Kohlenwasserstoffen die Höhe der $NO_2$-Konzentration bzw. das Konzentrationsverhältnis von $NO_2$ und NO Einfluss auf den Ablauf der maßgeblichen Reaktionen. Über eine Ermittlung der $NO_2$-Konzentration und/oder eines Konzentrationsverhältnisses von $NO_2$ und NO kann daher die Rußbeladung eines Partikelfilters oder auch eine Wirksamkeit eines SCR-Abgasnachbehandlungsverfahrens ermittelt bzw. modelliert oder Kennzahlen eines implementierten Reaktionsmodells adaptiert und so der Betrieb eines entsprechenden Abgasnachbehandlungssystems verbessert werden.

Bei dem Stickoxidsensor kann es sich um einen Sensor bekannter Bauart, beispielsweise auf Festelektrolytbasis oder um einen Halbleitersensor handeln. Entsprechende Sensoren sind beispielsweise in der DE 198 19 204 C1, der DE 43 34 672 A1, der EP 0 820 799 A2 oder der EP 0 723 662 B1 beschrieben. Erfindungsgemäß wird ein Stickoxidsensor eingesetzt, der ein Stickoxid-Ausgangssignal bereitstellt, welches mit einer durch die Summe der Konzentrationen von NO und $NO_2$ repräsentierten NOx-Gesamtkonzentration korreliert. Dieses Stickoxid-Ausgangssignal ist erfindungsgemäß das einzige in Bezug auf ein Stickoxid relevante Ausgangssignal. Dies schließt jedoch nicht aus, dass der Stickoxidsensor ein oder mehrere weiter Ausgangssignale zur Verfügung stellen kann, welche mit der Konzentration einer anderen Abgaskomponente wie z.B. Sauerstoff oder eines Abgaszustandsparameters wie beispielsweise der Abgastemperatur korrelieren.

In einer Ausgestaltung des efindungsgemäßen Verfahrens werden als erster und zweiter Stickoxidsensor Stickoxidsensoren gleicher Bauart eingesetzt. Auf diese Weise ist der Bauteilaufwand verringert und es lassen sich Kostenvorteile erzielen.

In einer weiteren vorteilhaften Ausgestaltung der Erfindung werden als erster und zweiter Stickoxidsensor Stickoxidsensoren eingesetzt, welche in Bezug auf NO und $NO_2$ unterschiedliche Stickoxid-Ausgangssignalkennlinien aufweisen. Durch eine zumindest in gewissen Konzentrationsbereichen unterschiedliche Empfindlichkeit der Stickoxidsensoren in Bezug auf NO und $NO_2$ ist eine genauere Ermittlung der $NO_2$-Konzentration bzw. des Konzentrationsverhältnisses von $NO_2$ und NO ermöglicht. Dabei ist es besonders vorteilhaft, wenn in weiterer Ausgestaltung des Verfahrens die Stickoxid-Ausgangssignalkennlinien des ersten Stickoxidsensors und des zweiten Stickoxidsensors annähernd linear mit unterschiedlichen Steigungen verlaufen. Ein zumindest annähernd linearer Kennlinienverlauf ermöglicht eine weitere Verbesserung der Genauigkeit des Verfahrens.

In weiterer Ausgestaltung des Verfahrens werden die $NO_2$-Konzentration und/oder das Konzentrationsverhältnis von $NO_2$ und NO in Abhängigkeit von einer Differenz oder eines Verhältnisses der Stickoxid-Ausgangssignale des ersten und des zweiten Stickoxidsensors ermittelt. Dadurch ist eine besonders einfache, jedoch ausreichend genaue Signalauswertung ermöglicht. Dabei wird typischerweise die Ausgangssignaldifferenz der Stickoxidsensoren wiederum mit dem Signalverhältnis korrelieren, sodass diese beiden Rechengrößen ineinander umgerechnet werden können. Analog wird typischerweise das Konzentrationsverhältnis von $NO_2$ und NO in einen hierzu proportionalen $NO_2$-Anteil der NOx-Konzentration umgerechnet werden können.

Besonders zuverlässige Ergebnisse können erzielt werden, wenn in weiterer Ausgestaltung des Verfahrens die $NO_2$-Konzentration und/oder das Konzentrationsverhältnis von $NO_2$ und NO am Ort des zweiten Stickoxidsensors ausgehend von einer vorgegebenen NOx-Gesamtkonzentration und/oder einer vorgegebenen $NO_2$-Konzentration am Ort des ersten Stickoxidsensors ermittelt werden.

In dem erfindungsgemäßen Verfahrens wird aus der ermittelten $NO_2$-Konzentration und/oder des Konzentrationsverhältnisses von $NO_2$ und NO eine Oxidationsrate von in einem dem zweiten Stickoxidsensor nachgeschalteten Partikelfilter zurückgehaltenem Ruß berechnet. Auf diese Weise ist eine verbesserte Modellierung einer zeitlich veränderlichen Rußbeladung des im Abgasstrang angeordneten Partikelfilters ermöglicht. Bei bekannter Rußabscheidewirkung des Partikelfilters und beispielsweise aus Betriebskennfeldern ermittelter Rußemission eines entsprechenden Verbrennungsmotors ist durch laufend durchgeführte Bilanzierung von im Partikelfilter ankommender und abgeschiedener Rußmenge und durch Oxidation mit $NO_2$ entfernter Rußmenge die aktuell im Partikelfilter angesammelte Rußmenge ermittelbar. Somit lassen sich Zeitpunkte, an denen eine zwangsweise, durch thermisch initiierten Rußabbrand bewirkte Partikelfilterregeneration durchgeführt werden soll, in vorteilhafter Weise festlegen. Bei einer seriellen Kombination von Oxidationskatalysator und nachgeschaltetem Partikelfilter im Abgasstrang ist hierzu bevorzugt der erste Stickoxidsensor in Abgasströmungsrichtung unmittelbar vor dem Oxidationskatalysator und der zweite Stickoxidsensor zwischen dem Oxidationskatalysator und dem Partikelfilter angeordnet.

In einer weiteren vorteilhaften Ausgestaltung des Verfahrens wird aus der ermittelten $NO_2$-Konzentration eine alterungsbedingte Wirkungsgradverschlechterung des Abgasbehandlungselements berechnet. Die Fähigkeit, eine Oxidation von NO zu $NO_2$ zu katalysieren, ist in vielen Fällen ein Indikator in Bezug auf eine alterungsbedingte Schädigung von oxidationskatalytisch wirksamen Abgasbehandlungselementen. Beispielsweise neigen für eine Oxidationswirkung maßgebliche Platinpartikel einer Beschichtung eines oxidationskatalytisch wirksamen Abgasbehandlungselements infolge thermischer Beanspruchung zur Agglomeration, mit der Folge einer verschlechterten katalytischen Wirkung. Mittels der erfindungsgemäßen Ermittlung einer $NO_2$-Konzentration und/oder eines Konzentrationsverhältnisses von $NO_2$ und NO ausgangsseitig des Abgasbehandlungselements lässt sich daher durch Vergleich auf die entsprechenden Werte vor diesem der Alterungszustand des Abgasbehandlungselements ermitteln. Damit ist eine zeitliche Anpassung von Steuergrößen und Prozessparametern eines Abgasnachbehandlungsprozesses ermöglicht, falls eine in Bezug auf $NO_2$ relevante alterungsbedingte Veränderung festgestellt wird.

In weiterer Ausgestaltung des Verfahrens wird aus der ermittelten $NO_2$-Konzentration und/oder des Konzentrationsverhältnisses von $NO_2$ und NO eine Stickoxidreduktionseffizienz eines dem zweiten Stickoxidsensor nachgeschalteten Stickoxidreduktionskatalysators berechnet. In einer besonders bevorzugten Variante werden die $NO_2$-Konzentration und/oder das Konzentrationsverhältnis von $NO_2$ und NO für in einen Stickoxid-SCR-Katalysator oder in einen Stickoxid-Speicherkatalysator einströmendes Abgas ermittelt. Bei dem diesem Katalysator vorgeschalteten oxidationskatalytisch wirksamen Abgasbehandlungselement mit Fähigkeit zur Umsetzung von NO zu $NO_2$ kann es sich beispielsweise um einen Oxidationskatalysator, einen Dreiwegekatalysator oder um einen katalytisch beschichteten Partikelfilter handeln. Die berechnete Stickoxidreduktionseffizienz ermöglicht wiederum eine geeignete Einstellung von Betriebsparametern für den Stickoxid-SCR-Katalysator oder Stickoxid-Speicherkatalysator. Beispielsweise kann vorgesehen sein, eine Einspeicherdauer von NOx oder eine NOx-Regenerationsdauer für den Stickoxid-Speicherkatalysator in Abhängigkeit von der Stickoxidreduktionseffizienz festzulegen. Für einen Stickoxid-SCR-Katalysator lassen sich in Abhängigkeit von der Stickoxidreduktionseffizienz eine Ammoniak- Harnstoff- oder Kohlenwasserstoffzufuhrrate festlegen. Ferner kann vorgesehen sein, in Abhängigkeit von der Stickoxidreduktionseffizienz Betriebsparameter der Verbrennungseinrichtung mit Blick auf eine geänderte NOx-Rohemission festzulegen. Bei einer als direkt einspritzenden Dieselmotor ausgebildeten Verbrennungseinrichtung kann eine Einstellung von Zeitpunkten für eine Vor-, Haupt-, oder Nacheinspritzung von Kraftstoff in die Zylinder des Motors in Abhängigkeit von der berechneten Stickoxidreduktionseffizienz vorgesehen sein. Es kann selbstverständlich vorgesehen sein, die genannten Maßnahmen und Einstellungen zusätzlich oder alternativ in Abhängigkeit von einem gegebenenfalls ermittelten Alterungszustand eines der Katalysatoren oder eines zusätzlich oder alternativ im Abgasstrang vorhandenen Abgasbehandlungselements vorzunehmen.

Vorteilhafte Ausführungsformen der Erfindung sind nachfolgend unter Bezug auf eine Zeichnung beschrieben. Dabei sind die vorstehend genannten und nachfolgend noch zu erläuternden Merkmale nicht nur in der jeweils angegebenen Merkmalskombination, sondern auch in anderen Kombinationen oder in Alleinstellung verwendbar, ohne den Rahmen der vorliegenden Erfindung zu verlassen.

Dabei zeigen:

Fig. 1 Eine in einem Abgasstrang eines Kraftfahrzeugs angeordnete Abgasbehandlungskomponente mit vor- und nachgeschaltetem Stickoxidsensor,

Fig. 2 ein Kennliniendiagramm zur Verdeutlichung einer Ausgangssignalcharakteristik für einen typischerweise eingesetzten Stickoxidsensor und

Fig. 3 ein Diagramm zur Verdeutlichung einer durch Verknüpfung von Ausgangssignalen von zwei Stickoxidsensoren erhaltenen Information.

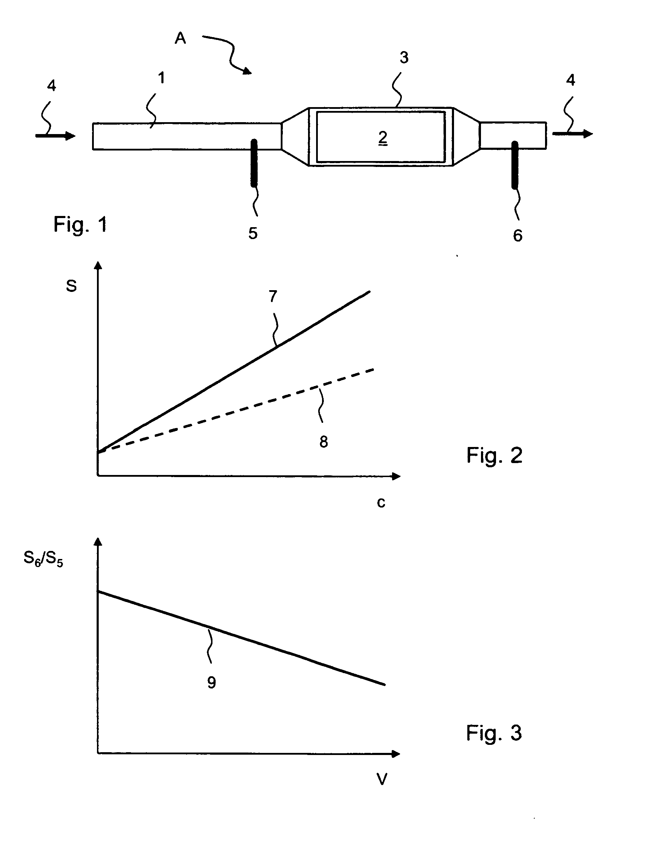

In Fig. 1 ist grob schematisch ein Ausschnitt A aus einem Abgasnachbehandlungssystem einer Kraftfahrzeug-Brennkraftmaschine dargestellt. Unter Beschränkung auf hier vorrangig interessierende Bestandteile zeigt Fig. 1 ein Abgasbehandlungselement 2, das in einem Abgasstrang 1 der nicht dargestellten Brennkraftmaschine angeordnet ist.

Das Abgasbehandlungselement 2 ist in einem Gehäuse 3 untergebracht und wird beim Betrieb der Brennkraftmaschine entsprechend den Pfeilen 4 vom Abgas der Brennkraftmaschine durch- und/oder umströmt. Stromauf und stromab des Abgasbehandlungselements 2 sind im Abgasstrang 1 ein erster Stickoxidsensor 5 und ein zweiter Stickoxidsensor 6 angeordnet. Die Stickoxidsensoren 5, 6 weisen eine Empfindlichkeit gegenüber NO und $NO_2$ auf und stellen jeweils ein entsprechendes, mit einer Stickoxidgesamtkonzentration korrelierendes Stickoxid-Ausgangssignal bereit, worauf weiter unten näher eingegangen wird. Bevorzugt handelt es sich bei den Stickoxidsensoren 5, 6 um solche gleicher Bauart und mit gleichem sensorischen Verhalten. Es können jedoch auch Stickoxidsensoren 5, 6 unterschiedlicher Bauart und mit unterschiedlichem sensorischen Verhalten eingesetzt werden. Zur Auswertung der Stickoxid-Ausgangssignale sind die Stickoxidsensoren 5, 6 in nicht dargestellter Weise an ein elektronisches Steuergerät mit Rechnerfunktion angeschlossen.

Das Abgasbehandlungselement 2 weist die Eigenschaft auf, zur Umsetzung von NO zu $NO_2$ befähigt zu sein. Hierfür kommt eine Vielzahl unterschiedlicher Komponenten wie beispielsweise ein Oxidationskatalysator oder gegebenenfalls eine andere Komponente in Betracht. Dem Abgasbehandlungselement 2 ist ein Partikelfilter nachgeschaltet und es können weitere Abgasbehandlungskomponenten oder Bauteile wie beispielsweise ein SCR-, Dreiwege-, Stickoxidspeicher- oder Oxidationskatalysator, weitere Abgassensoren, wie beispielsweise Lambdasensoren oder Temperaturfühler vor- und/oder nachgeschaltet sein, was nicht gesondert dargestellt ist.

Nachfolgend wird ohne Einschränkung der Allgemeinheit davon ausgegangen, dass es sich bei dem Abgasbehandlungselement 2 um einen Oxidationskatalysator handelt, welcher als erstes von Abgas durchströmtes Abgasbehandlungselement nahe der Brennkraftmaschine im Abgasstrang 1 angeordnet ist und daher mit Abgas beaufschlagt wird, welches gegenüber seiner ursprünglichen Zusammensetzung lediglich vernachlässigbare oder keine Veränderungen erfahren hat.

Nachfolgend wird näher darauf eingegangen, auf welche Weise zur Beurteilung oder Steuerung bzw. Regelung eines im Abgasstrang 1 allgemein vorgesehenen Abgasreinigungsprozesses, welcher bei oder nach Passieren des Abgasbehandlungselements 2 abgelaufen ist oder abläuft, durch Vergleich der Stickoxid-Ausgangssignale der Stickoxidsensoren 5, 6 die $NO_2$-Konzentration und/oder das Konzentrationsverhältnis von $NO_2$ und NO insbesondere am Ort des zweiten Stickoxidsensors 6 ermittelt werden kann. Grundlage hierfür bildet die beispielhaft nachfolgend vorausgesetzte Tatsache, dass die Stickoxidsensoren 5, 6 jeweils unterschiedliche Empfindlichkeiten für NO und $NO_2$ aufwiesen. Die entsprechenden Kennlinien können prinzipiell nahezu beliebig ausgeprägt sein.

Unter Bezug auf Fig. 2 wird nachfolgend ohne Einschränkung der Allgemeinheit eine vorteilhafte Vorgehensweise erläutert, welche bei annähernd linearen Stickoxid-Ausgangssignalkennlinien des ersten Stickoxidsensors 5 und des zweiten Stickoxidsensors 6 bevorzugt angewendet wird. Im Diagramm von Fig. 2 sind die Abhängigkeiten $S = S(c)$ der Stickoxid-Ausgangssignale S der Stickoxidsensoren 5, 6 von der Konzentration c von NO bzw. $NO_2$ dargestellt. Dabei sei beispielhaft die mit 7 bezeichnete Kennlinie die für NO maßgebende Kennlinie, während die mit 8 bezeichnete Kennlinie geringerer Steigung für $NO_2$ maßgebend sei. Der umgekehrte Fall ist natürlich ebenso möglich. Ein von einem der Stickoxidsensoren 5, 6 bereitgestelltes Stickoxid-Ausgangssignal S besitzt daher im Allgemeinen einen ersten Anteil, welcher von der NO-Konzentration und einen zweiten Anteil, der von der $NO_2$-Konzentration im Abgas verursacht ist. Dabei kann zunächst nicht beurteilt werden, in welchem Ausmaß jedes dieser Anteile zum mit der Stickoxidgesamtkonzentration korrelierenden Stickoxid-Ausgangssignal S beiträgt. Infolge des erfindungsgemäß vorgenommenen Vergleichs der Stickoxid-Ausgangssignale S der Stickoxidsensoren 5, 6 kann jedoch insbesondere für den Fall einer vorgegebenen Randbedingung die $NO_2$-Konzentration und/oder das Konzentrationsverhältnis von $NO_2$ und NO am Ort des zweiten Stickoxidsensors 6 ermittelt werden. In einfachen Fällen ist ein Vergleich in Form einer Differenz- oder Verhältnisbildung ausreichend. Der Vergleich kann jedoch auch komplexere, beispielsweise nichtlineare Rechen- oder Näherungsverfahren oder dergleichen beinhalten. Wird beispielhaft davon ausgegangen, dass die $NO_2$-Konzentration im Abgas an der Stelle des ersten Stickoxidsensors 5 vernachlässigbar ist, was z.B. bei einer brennkraftmaschinennahen Anordnung des Abgasbehandlungselements 2 regelmäßig der Fall ist, so ergeben sich folgende Zusammenhänge, wenn die Stickoxidgesamtkonzentration im Abgas durch das Abgasbehandlungselement 2 nicht beeinflusst wird und Stickoxidsensoren 5, 6 mit gleichen Stickoxid-Ausgangssignalkennlinien 7, 8 eingesetzt werden:

$$c_{5NO} * m_7 = S_5 \qquad\qquad (1)$$

$$c_{6NO} * m_7 + c_{6NO2} * m_8 = S_6 \qquad (2)$$

$$c_{5NO} - c_{6NO} - c_{6NO2} = 0 \qquad (3).$$

Dabei bezeichnen $c_{5NO}$, $c_{6NO}$, $c_{6NO2}$ die Konzentrationen c von NO und $NO_2$ an den Orten der Stickoxidsensoren 5, 6, $m_7$, $m_8$ Steigungen der Stickoxid-Ausgangssignalkennlinien 7, 8 und $S_5$, $S_6$ die Stickoxid-Ausgangssignale der Stickoxidsensoren 5, 6.

Aus den Gleichunge (1) bis (3) ergibt sich zwischen der $NO_2$-Konzentration $c_{6NO2}$ am Ort des zweiten Stickoxidsensors 6 und der Differenz der Stickoxid-Ausgangssignale S der Stickoxidsensoren 5, 6 der folgende lineare Zusammenhang

$$c_{NO2} = (S_6 - S_5)/(m_8 - m_7) \qquad (4).$$

Mit modifizierten Rechnungsgängen lassen sich auch andere Zusammenhänge herleiten. Beispielhaft zeigt Fig. 3 ein Diagramm mit einer durch die Kurve 9 wiedergegebenen Abhängigkeit des Verhältnisses $S_6/S_5$ der Stickoxid-Ausgangssignale $S_5$, $S_6$ der Stickoxidsensoren 5, 6 von einem Verhältnis V von $NO_2$-Konzentration und NOx-Konzentration am Ort des zweiten Stickoxidsensors 6. Unter den oben genannten Voraussetzungen ergibt sich ebenfalls ein linearer Zusammenhang. Analog lässt sich ein Verhältnis der Konzentrationen von $NO_2$ und NO in Abhängigkeit einer Verknüpfung der Stickoxid-Ausgangssignale $S_5$, $S_6$ der Stickoxidsensoren 5, 6 ermitteln. Vorzugsweise werden die genannten Größen in Echtzeit aus den von den Stickoxidsensoren 5, 6 bereitgestellten Stickoxid-Ausgangssignalen $S_5$, $S_6$ ermittelt. Vorteilhaft ist dabei ein Rückgriff auf vorab ermittelte und in Form von Wertetabellen oder Kennlinien entsprechend Fig. 2 bereitgestellten Daten. Vorteilhaft ist weiterhin ein Plausibilitätsabgleich mit abgespeicherten und vorgehaltenen Daten für die NOx-Rohemission der Brennkraftmaschine für verschiedene Betriebspunkte oder -Bereiche.

Es versteht sich, dass die oben lediglich beispielhaft genannten Berechnungsverfahren in modifizierter Form anwendbar sind, wenn von anderen vorgegebenen Randbedingungen ausgegangen wird.

Es kann auch vorgesehen sein, die in Fig. 1 dargestellte Anordnung zu modifizieren. Beispielsweise können als Abgasbehandlungselement 2 zwei oder mehrere separate Komponenten vorgesehen sein. Diese können seriell hintereinander oder parallel zueinander angeordnet sein. Dabei können auch drei oder mehr Stickoxidsensoren eingesetzt werden, wodurch eine Mehrzahl von $NO_2$-Konzentrationen und/oder Konzentrationsverhältnissen von $NO_2$ und NO an verschiedenen Orten im Abgasstrang 1 ermittelt werden können.

Die in einer entsprechenden erfindungsgemäßen Vorgehensweise ermittelten Daten betreffend eine $NO_2$-Konzentration und/oder ein Konzentrationsverhältnis von $NO_2$ und NO lassen sich in vielfältiger Weise weiterverarbeiten. Dadurch können weitere Informationen betreffend den Zustand und die Wirksamkeit von im Abgasstrang 1 angeordneten weiteren Abgasreinigungskomponenten ermittelt und/oder der Betrieb von Abgasreinigungskomponenten gesteuert oder geregelt werden.

Erfindungsgemäß ist es vorgesehen, dass aus der ermittelten Stickstoffdioxidkonzentration und/oder dem Konzentrationsverhältnis von Stickstoffdioxid und Stickstoffmonoxid eine Oxidationsrate von in einem dem zweiten Stickoxidsensor (6) nachgeschaltetem Partikelfilter zurückgehaltenem Ruß berechnet wird.

Zusätzlich lässt sich durch Vergleich mit Referenzwerten der Alterungszustand oder eine Schädigung eines Abgasbehandlungselements 2 insbesondere bei vorgegebenen Betriebsbedingungen ermitteln. Dies kann insbesondere für ein als Oxidationskatalysator ausgebildetes Abgasbehandlungselement 2 vorgesehen sein, da die Fähigkeit zur NO-Oxidation bei einer alterungsbedingten Schädigung bei üblichen Oxidationskatalysatoren nachlässt.

Ferner kann es zusätzlich vorgesehen sein, die erfindungsgemäß ermittelten Daten zur $NO_2$-Konzentration im Abgas zur Steuerung bzw. Regelung des Betriebs eines SCR-Katalysators zu nutzen. Hierfür ist es vorteilhaft, eine üblicherweise gegebene Abhängigkeit eines NOx-Reduktionswirkungsgrads von der $NO_2$-Konzentration oder vom Verhältnis von $NO_2$ und NO in Form eines Kennfelds oder einer Kennlinie vorzuhalten und eine Reduktionsmittelzufuhr in Abhängigkeit der aktuellen $NO_2$-Konzentrationswerte bzw. des davon abhängigen Katalysatorwirkungsgrads einzustellen.

Ebenfalls zusätzlich kann vorgesehen sein, eine $NO_2$-Konzentration im Abgas unmittelbar vor dessen Abgabe an die Umgebung (tail-pipe Emission) mittels des erfindungsgemäßen Verfahrens zu überwachen. Zweckmäßig ist es hierfür, Stickoxidsensoren 5, 6 eingangs- und ausgangsseitig eines in Abgasströmungsrichtung 4 endseitig im Abgassystem vorgesehenen Abgasbehandlungselements 2 vorzusehen.

**Patentansprüche**

1. Verfahren zur Berechnung einer Rußoxidationsrate, wobei eine Stickstoffdioxidkonzentration und/oder ein Konzentrationsverhältnis von Stickstoffdioxid und Stickstoffmonoxid in einem Abgasstrang (1) einer Brennkraftmaschine mittels eines in Bezug auf Stickstoffmonoxid und Stickstoffdioxid empfindlichen Stickoxidsensors (5; 6) ermittelt wird, welcher als einziges in Bezug auf Stickoxide relevantes Ausgangssional ein Stickoxid-Ausgangssignal (S) bereitstellt, das mit einer durch die Summe der Konzentrationen von Stickstoffmonoxid und Stickstoffdioxid repräsentierten Stickoxidgesamtkonzentration korreliert, wobei zur Ermittlung der Stickstoffdioxidkonzentration und/oder des Konzentrationsverhältnisses von Stickstoffdioxid und Stickstoffmonoxid das Stickoxid-Ausgangssignal (S) eines ersten Stickoxidsensors (5), der stromauf eines im Abgasstrang (1) angeordneten oxidationskatalytisch wirksamen Abgasbehandlungselements (2) mit Fähigkeit zur Umsetzung von Stickstoffmonoxid zu Stickstoffdioxid angeordnet ist, mit dem Stickoxid-Ausgangssignal (S) eines zweiten Stickoxidsensors (6), der stromab des oxidationskatalytisch wirksamen Abgasbehandlungselements (2) und angeordnet ist, verglichen wird und aus der ermittelten Stickstoffdioxidkonzentration und/oder aus dem ermittelten Konzentrationsverhältnis von Stickstoffdioxid und Stickstoffmonoxid die Rußoxidationsrate von in einem dem zweiten Stickoxidsensor (6) nachgeschalteten Partikelfilter zurückgehaltenem Ruß berechnet wird.

2. Verfahren nach Anspruch 1,
   **dadurch gekennzeichnet, dass**
   unter Berücksichtigung der ermittelten Stickstoffdioxidkonzentration und/oder des ermittelten Konzentrationsverhältnisses von Stickstoffdioxid und Stickstoffmonoxid in einem Modell eine zeitlich veränderliche Rußbeladung des Partikelfilters ermittelt wird.

3. Verfahren nach Anspruch 1 oder 2,
   **dadurch gekennzeichnet, dass**
   basierend auf der ermittelten Stickstoffdioxidkonzentration und/oder des ermittelten Konzentrationsverhältnisses von Stickstoffdioxid und Stickstoffmonoxid Zeitpunkte, an denen eine zwangsweise, durch thermischen Rußabbrand bewirkte Regeneration des Partikelfiters durchgeführt werden soll, festgelegt werden..

4. Verfahren nach einem der Ansprüche 1 bis 3,
   **dadurch gekennzeichnet, dass**
   als erster Stickoxidsensor (5) und zweiter Stickoxidsensor (6) Stickoxidsensoren gleicher Bauart eingesetzt werden.

5. Verfahren nach einem der Ansprüche 1 bis 4,
   **dadurch gekennzeichnet, dass**
   als erster Stickoxidsensor (5) und zweiter Stickoxidsensor (6) Stickoxidsensoren eingesetzt werden, welche in Bezug auf Stickstoffmonoxid und Stickstoffdioxid unterschiedliche Stickoxid-Ausgangssignalkennlinien aufweisen.

6. Verfahren nach einem der Ansprüche 1 bis 5,
   **dadurch gekennzeichnet, dass**
   die Stickoxid-Ausgangssignalkennlinien des ersten Stickoxidsensors (5) und des zweiten Stickoxidsensors (6) annähernd linear mit unterschiedlichen Steigungen verlaufen.

7. Verfahren nach einem der Ansprüche 1 bis 6,
   **dadurch gekennzeichnet, dass**
   die Stickstoffdioxidkonzentration und/oder das Konzentrationsverhältnis von Stickstoffdioxid und Stickstoffmonoxid in Abhängigkeit von einer Differenz oder eines Verhältnisses der Stickoxid-Ausgangssignale (S) des ersten und des zweiten Stickoxidsensors (5, 6) ermittelt werden.

8. Verfahren nach einem der Ansprüche 1 bis 7,
   **dadurch gekennzeichnet, dass**
   die Stickstoffdioxidkonzentration und/oder das Konzentrationsverhältnis von Stickstoffdioxid und Stickstoffmonoxid am Ort des zweiten Stickoxidsensors (6) ausgehend von einer vorgegebenen Stickoxidgesamtkonzentration und/oder einer vorgegebenen Stickstoffdioxidkonzentration am Ort des ersten Stickoxidsensors (5) ermittelt wird.

9. Verfahren nach einem der Ansprüche 1 bis 8,
   **dadurch gekennzeichnet, dass**
   aus der ermittelten Stickstoffdioxidkonzentration und/oder des Konzentrationsverhältnisses von Stickstoffdioxid und Stickstoffmonoxid eine alterungsbedingte Wirkungsgradverschlechterung des Abgasbehandlungselements (2) berechnet wird.

10. Verfahren nach einem der Ansprüche 1 bis 9,
   **dadurch gekennzeichnet, dass**
   aus der ermittelten Stickstoffdioxidkonzentration und/oder des Konzentrationsverhältnisses von Stickstoffdioxid und Stickstoffmonoxid eine Stickoxidreduktionseffizienz eines dem zweiten Stickoxidsensor (6) nachgeschalteten Stickoxidreduktionskatalysators berechnet wird.

**Claims**

1. Method for calculating a particulate oxidation rate, wherein a nitrogen dioxide concentration and/or a concentration ratio between nitrogen dioxide and nitric oxide in an exhaust train (1) of an internal combustion engine is determined by means of a nitrogen oxide sensor (5; 6) sensitive to nitric oxide and nitrogen dioxide, which sensor provides a nitrogen oxide output signal (S) as an only output signal relevant to nitrogen oxides, which output signal correlates to a total nitrogen oxide concentration represented by the sum of the concentrations of nitric oxide and nitrogen dioxide, wherein, for the determination of the nitrogen dioxide concentration and/or the concentration ratio between nitrogen dioxide and nitric oxide, the nitrogen oxide output signal (S) of a first nitrogen oxide sensor (5) positioned upstream of an exhaust treatment element (2) located in the exhaust train (1), acting in the manner of an oxidising catalyst and capable of converting nitric oxide into nitrogen dioxide is compared to the nitrogen oxide output signal (S) of a second nitrogen oxide sensor (6) located downstream of the exhaust treatment element (2) acting in the manner of an oxidising catalyst, and wherein the particulate oxidation rate of particulates retained in a particulate filter provided downstream of the second nitrogen oxide sensor (6) is calculated from the detected nitrogen dioxide concentration and/or from the detected concentration ratio between nitrogen dioxide and nitric oxide.

2. Method according to claim 1,
   **characterised in that**,
   taking into account the detected nitrogen dioxide concentration and/or the detected concentration ratio between nitrogen dioxide and nitric oxide, a particulate loading of the particulate filter which is variable in time is determined in a model.

3. Method according to claim 1 or 2,
   **characterised in that**,
   based on the detected nitrogen dioxide concentration and/or the detected concentration ratio between nitrogen dioxide and nitric oxide, times at which the particulate filter has to be regenerated by forced thermal particulate depletion are determined.

4. Method according to any of claims 1 to 3,
   **characterised in that**
   nitrogen oxide sensors of the same design are used as first nitrogen oxide sensor (5) and as second nitrogen oxide sensor (6).

5. Method according to any of claims 1 to 4,
   **characterised in that**
   nitrogen oxide sensors having different nitrogen oxide output signal characteristics in terms of nitric oxide and nitrogen dioxide are used as first nitrogen oxide sensor (5) and as second nitrogen oxide sensor (6).

6. Method according to any of claims 1 to 5,
   **characterised in that**
   the nitrogen oxide output signal characteristics of the first nitrogen oxide sensor (5) and the second nitrogen oxide sensor (6) are approximately linear with different slopes.

7. Method according to any of claims 1 to 6,
   **characterised in that**
   the nitrogen dioxide concentration and/or the concentration ratio between nitrogen dioxide and nitric oxide is/are determined as a function of a difference or a ratio of the nitrogen oxide output signals (S) of the first and second nitrogen oxide sensors (5, 6).

**8.** Method according to any of claims 1 to 7,
**characterised in that**
the nitrogen dioxide concentration and/or the concentration ratio between nitrogen dioxide and nitric oxide is/are determined at the point of the second nitrogen oxide sensor (6) starting from a predetermined total nitrogen oxide concentration and/or a predetermined nitrogen dioxide concentration at the point of the first nitrogen oxide sensor (5).

**9.** Method according to any of claims 1 to 8,
**characterised in that**,
from the detected nitrogen dioxide concentration and/or the detected concentration ratio between nitrogen dioxide and nitric oxide, an ageing-related efficiency deterioration of the exhaust treatment element (2) is calculated.

**10.** Method according to any of claims 1 to 8,
**characterised in that**,
from the detected nitrogen dioxide concentration and/or the detected concentration ratio between nitrogen dioxide and nitric oxide, a nitrogen oxide reduction efficiency of a nitrogen oxide reduction catalyst provided downstream of the second nitrogen oxide sensor (6) is calculated.

**Revendications**

**1.** Procédé pour calculer le taux d'oxydation de la suie, une concentration en dioxyde d'azote et / ou un rapport de concentration de dioxyde d'azote et monoxyde d'azote étant déterminé dans une ligne d'échappement (1) d'un moteur à combustion interne au moyen d'un capteur d'oxyde d'azote (5, 6) sensible au monoxyde d'azote et au dioxyde d'azote, ledit capteur fournit un signal de sortie (S) d'oxyde d'azote en tant qu'unique signal de sortie pertinent par rapport à l'oxyde d'azote, qui est corrélé à une concentration totale en oxyde d'azote représentée par la somme des concentrations de monoxyde d'azote et de dioxyde d'azote, pour déterminer la concentration en dioxyde d'azote et / ou le rapport de concentration de dioxyde d'azote et de monoxyde d'azote, le signal de sortie d'oxyde d'azote (S) d'un premier capteur d'oxyde d'azote (5) qui est disposé en amont d'un élément (2) de traitement des gaz d'échappement à activité catalytique d'oxydation disposé dans la ligne d'échappement (1) et capable de convertir le monoxyde d'azote en dioxyde d'azote, étant comparé au signal de sortie d'oxyde d'azote (S) d'un deuxième capteur d'oxyde d'azote (6) qui est disposé en aval de l'élément (2) de traitement des gaz d'échappement à activité catalytique d'oxydation et à partir de la concentration en dioxyde d'azote déterminée et / ou à partir du rapport de concentration déterminé de dioxyde d'azote et de monoxyde d'azote, le taux d'oxydation de la suite retenue dans un filtre à particules monté en aval du deuxième capteur d'oxyde d'azote (6) est calculé.

**2.** Procédé selon la revendication 1, **caractérisé en ce qu'**en tenant compte de la concentration en dioxyde d'azote déterminée et / ou du rapport de concentration déterminé de dioxyde d'azote et de monoxyde d'azote, une charge de suite du filtre à particules variant dans le temps est définie sous forme d'un modèle.

**3.** Système d'usinage selon la revendication 1 ou 2, **caractérisé en ce qu'**il est défini selon la concentration déterminée de dioxyde d'azote et / ou du rapport de concentration déterminé du dioxyde d'azote et du monoxyde d'azote des moments où il est effectué une régénération forcée du filtre à particules provoquée par la combustion thermique de la suie.

**4.** Procédé selon la revendication 1, **caractérisé en ce que** des capteurs d'oxyde d'azote de même type que le premier capteur d'oxyde d'azote (5) et le deuxième capteur d'oxyde d'azote (6) sont utilisés.

**5.** Procédé selon l'une quelconque des revendications de 1 à 4, **caractérisé en ce qu'**il est utilisé des capteurs d'oxyde d'azote comme le premier capteur d'oxyde d'azote (5) et le deuxième capteur d'oxyde d'azote (6), qui présentent par rapport au monoxyde d'azote et au dioxyde d'azote des courbes caractéristiques de signal de sortie d'oxyde d'azote différents.

**6.** Procédé selon l'une quelconque des revendications de 1 à 5, **caractérisé en ce que** les courbes caractéristiques de signal de sortie d'oxyde d'azote du premier capteur d'oxyde d'azote (5) et du deuxième capteur d'oxyde d'azote (6) sont approximativement linéaires avec différentes progressions.

**7.** Procédé selon l'une quelconque des revendications 1 à 6, **caractérisée en ce que** la concentration en dioxyde d'azote et / ou le rapport de concentration en dioxyde d'azote et monoxyde d'azote est défini(e) en fonction d'une différence ou d'un rapport des signaux de sortie d'oxyde d'azote (S) du premier et du deuxième capteurs d'oxyde d'azote (5, 6).

8.  Procédé selon l'une quelconque des revendications de 1 à 7, **caractérisé en ce que** la concentration de dioxyde d'azote et / ou le rapport de concentration de dioxyde d'azote et de monoxyde d'azote au niveau du deuxième capteur d'oxyde d'azote (6) est défini(e) à partir d'une concentration totale d'oxyde d'azote prédéfinie et / ou d'une concentration de dioxyde d'azote prédéfinie au niveau du premier capteur d'oxyde d'azote (5).

9.  Procédé selon l'une quelconque des revendications de 1 à 8, **caractérisé en ce qu'**à partir de la concentration déterminée de dioxyde d'azote et / ou du rapport de concentration en dioxyde d'azote et en monoxyde d'azote, il est calculé une réduction du rendement de l'élément de traitement des gaz d'échappement (2) dû au vieillissement.

10. Procédé selon l'une quelconque des revendications de 1 à 9, **caractérisé en ce qu'**à partir de la concentration déterminée de dioxyde d'azote et / ou du rapport de concentration du dioxyde d'azote et du monoxyde d'azote, il est calculé une amélioration de la réduction d'oxyde d'azote d'un catalyseur de réduction d'oxyde d'azote monté en aval du deuxième capteur d'oxyde d'azote (6).

Fig. 1

Fig. 2

Fig. 3

**IN DER BESCHREIBUNG AUFGEFÜHRTE DOKUMENTE**

**In der Beschreibung aufgeführte Patentdokumente**

- DE 102005049655 A1
- EP 0869359 A2
- FR 2864146 A1
- DE 4402850 A1
- DE 19819204 C1
- DE 4334672 A1
- EP 0820799 A2
- EP 0723662 B1

**In der Beschreibung aufgeführte Nicht-Patentliteratur**

- **KANDYLAS et al.** Diesel Soot Oxidation with NO2: Engine Experiments and Simulations. *Ind. Eng. Chem. Res.,* vol. 41 (22), 5372-5384